# EUROPÄISCHE PATENTSCHRIFT

(11) **EP 3 068 351 B1**
(45) Veröffentlichungstag und Bekanntmachung des Hinweises auf die Patenterteilung: **29.03.2017**
(21) Anmeldenummer: 15791506.7
(22) Anmeldetag: 10.09.2015
(51) Int. Cl.: A61F 5/01

(54) **ORTHESE**
ORTHOSIS
ORTHÈSE

(30) Priorität: 11.09.2014 AT 6932014
(43) Veröffentlichungstag der Anmeldung: 21.09.2016
(73) Patentinhaber: Allomed Medizintechnik GmbH, 2320 Schwechat (AT); Roscher, Rupert, 6300 Wörgl (AT)
(72) Erfinder: ROSCHER, Rupert, 6300 Wörgl (AT); MILACEK, Walter, 2320 Schwechat (AT)
(74) Vertreter: Ellmeyer, Wolfgang
(86) Internationale Anmeldenummer: PCT/EP2015/070777
(87) Internationale Veröffentlichungsnummer: WO 2016/038167

(56) Entgegenhaltungen:
- WO-A1-98/29060
- DE-U1- 29 813 372
- US-A- 4 554 912
- US-A1- 2006 004 311
- US-B1- 6 602 215

## Beschreibung

Die vorliegende Erfindung betrifft eine neue Kombination aus Sprunggelenks- und Fußorthese.

### STAND DER TECHNIK

Orthesen sind medizinische Hilfsmittel, die zur Stabilisierung, Entlastung, Ruhigstellung, Führung oder Korrektur von Gliedmaßen oder des Rumpfes nach Verletzungen oder aufgrund von Fehlstellungen eingesetzt werden. Im Fußbereich werden Orthesen beispielsweise nach Verletzungen des Spunggelenks oder anderer Fußknochen oder -sehnen, sowie im Falle von Lähmungserscheinungen, z.B. als Folge von Traumata oder Schlaganfällen, verordnet.

Sprunggelenksorthesen umfassen mehrheitlich einen Schaft, der sich zumeist von der Sohle oder Ferse bis über den Knöchel erstreckt, mitunter ein sich über einen Teil der Fußsohle erstreckendes Sohlenelement, sowie einen oder mehrere Riemen oder Bänder zur Fixierung des Schafts am Fuß und/oder Unterschenkel, z.B. mittels Verschnürung oder Klettverschlüssen, oder auch in Form eines an beiden Seiten fix am Schaft befestigten elastischen Bandes.

Die Position und Länge dieser Bänder oder Riemen variieren stark in Abhängigkeit von der zu behandelnden Verletzung oder Fehlstellung. Beispielsweise existieren Ausführungsformen, die eine Verschnürung ähnlich jener eines Schuhs aufweisen, oder häufig sind zwei Bänder oder Riemen vorgesehen, von denen eines/-r um den Unterschenkel und ein zweites/-r um den Fuß herum geführt wird, die beide mitunter mittels Klettverschluss fixiert werden.

Nachteilig ist dabei, dass die wenigsten bekannten Ausführungsformen von Orthesen mehrere Funktionen in zufrieden stellender Weise erfüllen können, z.B. Stabilisierung des Sprunggelenks bei gleichzeitiger Unterstützung der Reklination, d.h. Zurückbiegen, des Fußes. Ziel der Erfindung war daher die Bereitstellung einer neuen Orthese, die dazu in der Lage ist.

US 6602215 B1 offenbart eine Sprunggelenkorthese gemäß dem Oberbegriff des Anspruchs 1.

### OFFENBARUNG DER ERFINDUNG

Dieses Ziel erreicht die Erfindung durch Bereitstellung einer Sprunggelenksorthese, die Folgendes umfasst: einen länglichen Schaft, der von der Ferse bis über den Knöchel verläuft und tibial (nach vorne hin) offen ist, ein mit dem Schaft verbundenes, die Fußsohle zumindest teilweise abdeckendes Sohlenelement, einen ersten Riemen zur Befestigung des Schafts am Unterschenkel und einen zweiten Riemen zur Befestigung des Sohlenelements am Fuß, und die dadurch gekennzeichnet ist, dass:
a) der Schaft und das Sohlenelement jeweils einen unbiegsamen Abschnitt und einen biegsamen Abschnitt umfassen,
   wobei der unbiegsame Schaftabschnitt sich von einer plantaren Position unterhalb der Ferse lateral über einen Knöchel hinaus aufwärts erstreckt und im Bereich seines proximalen Randes einen Teil eines ersten Klettverschlusses aufweist und der biegsame Schaftabschnitt sich kontralateral von der Ferse über den anderen Knöchel hinaus aufwärts erstreckt,
   wobei der unbiegsame Sohlenabschnitt sich von der Ferse bis zum Fußgewölbe erstreckt und der biegsame Sohlenabschnitt sich zumindest vom Fußgewölbe in Richtung der Zehen erstreckt;
b) der erste Riemen am distalen Rand des biegsamen Schaftabschnitts befestigt ist, an seinem Ende den zweiten Teil des ersten Klettverschlusses aufweist und ausreichende Länge besitzt, um die beiden Teile des ersten Klettverschlusses miteinander verbinden zu können;
c) der zweite Riemen am distalen Ende des unbiegsamen Sohlenabschnitts befestigt ist und zwei Teile eines zweiten Klettverschlusses umfasst,
   wobei ein Teil des zweiten Klettverschlusses am Ende des zweiten Riemens vorgesehen ist und der andere Teil in einem mittleren Bereich und auf der gegenüberliegenden Seite des zweiten Riemens vorgesehen ist,
   wobei der zweite Riemen ausreichende Länge aufweist, dass die beiden Teile des zweiten Klettverschlusses miteinander verbindbar sind, wenn der zweite Riemen quer über den Fußrücken und um beide Knöchel herum geführt wird; und
d) am distalen Ende des unbiegsamen Sohlenabschnitts auch ein dritter Riemen befestigt ist, der zwei Teile eines dritten Klettverschlusses umfasst,
wobei ein Teil des dritten Klettverschlusses am Ende des dritten Riemens vorgesehen ist und der andere Teil in einem mittleren Bereich und auf der gegenüberliegenden Seite des dritten Riemens vorgesehen ist,
wobei der dritte Riemen ausreichende Länge aufweist, dass die beiden Teile des dritten Klettverschlusses miteinander verbindbar sind, wenn der dritte Riemen um den Fuß herum geführt wird.

Eine solche Sprunggelenksorthese ist im Gegensatz zu Ausführungsformen gemäß dem Stand der Technik äußerst vielseitig bei diversen Krankheitsbildern, d.h. sowohl für Unfallchirurgie- und Orthopädie- als auch Neurologiepatienten einsetzbar, da sie gleichzeitig zur Stabilisierung des Sprunggelenks und zur Unterstützung der Reklination des Fußes dient und das Anziehen des Schuhs erleichtert. Sie erfüllt somit drei Funktionen, nämlich Sprunggelenksorthese, Fußorthese und Schuhanziehhilfe.

Wesentlich ist dafür in erster LInie, dass sowohl der Schaft als auch das Sohlenelement jeweils einen biegsamen und einen unbiegsamen Abschnitt aufweisen und die beiden unbiegsamen Abschnitte nicht, wie ansonsten üblich, einstückig oder starr miteinander verbunden sind. Weiters ist aber auch die Gegenwart dreier Riemen, von denen einer den Schaft am Unterschenkel fixiert, einer die Verbindung zwischen Fuß und Unterschenkel stabilisiert und einer die Sohle am Fuß fixiert, für die Wirkung der Erfindung maßgeblich.

Gemäß vorliegender Erfindung sind daher nicht die beiden unbiegsamen Abschnitte von Schaft und Sohle miteinander verbunden, sondern jeweils ein biegsamer mit einem unbiegsamen Abschnitt der beiden. Das heißt, es ist entweder der unbiegsame Sohlenabschnitt mit dem biegsamen Schaftabschnitt oder der unbiegsame Schaftabschnitt mit dem biegsamen Sohlenabschnitt verbunden, um zusammen die Orthese zu ergeben, aber ein Verkippen der Sohle relativ zum Schaft und damit eine Reklination des Fußes zu ermöglichen. Eine solche Verbindung ist vorzugsweise nicht im Bereich der Ferse, sondern plantar unterhalb der Fußwölbung vorgesehen, was für eine geringere Beanspruchung der Verbindung bei Verwendung der Orthese sorgt.

Zu diesem Zweck erstreckt sich in bevorzugten Ausführungsformen der biegsame Schaftabschnitt im Bereich der Ferse bis unter den unbiegsamen Schaftabschnitt und ist dort sowohl mit Letzterem fest verbunden, z.B. verklebt oder verschweißt, als auch einstückig mit zumindest einem Teil des biegsamen Sohlenabschnitts ausgeführt, d.h. der biegsame Schaftabschnitt geht nahtlos in den biegsamen Sohlenabschnitts über. Dieser Teil des biegsamen Sohlenabschnitts - oder auch der gesamte biegsame Sohlenabschnitt - ist seinerseits mit dem unbiegsamen Sohlenabschnitt verbunden, vorzugsweise verklebt oder vernietet, wodurch die Verkippbarkeit der unbiegsamen Abschnitte zueinander gewährleistet ist.

Weiters weist der unbiegsame Schaftabschnitt vorzugsweise eine solche Breite auf, dass er fibular (an der Rückseite) etwa den halben Unterschenkel abdeckt. Dies gewährleistet einerseits Stabilität der durch den ersten Riemen hergestellten Verbindung mit dem Unterschenkel, sorgt bei entsprechender Höhe der Orthese für einen erwünschten Gegendruck auf die Wade, engt aber die Muskulatur des Trägers der Orthese im distalen Bereich des Unterschenkels nicht übermäßig ein, was die Beweglichkeit des Fußgelenks bei Reklination unterstützt und allenfalls auch den Muskel-Wiederaufbau nach längerfristiger Ruhestellung des Beins fördert.

Andererseits weist der biegsame Schaftabschnitt vorzugsweise eine solche Breite auf, dass er tibial (an der Vorderseite des Beins) etwa den halben Unterschenkel abdeckt. Dadurch wird wiederum der Sitz des Schafts am Unterschenkel stabilisiert, zusätzlich aber dient der biegsame Schaftabschnitt so auch als Unterlage für den ersten Riemen, der ja quer über diesen Bereich bis zum Klettverschluss am proximalen Ende des unbiegsamen Schaftabschnitts geführt wird.

In weiteren bevorzugten Ausführungsformen der Erfindung ersteckt sich der biegsame Sohlenabschnitt bis zu den Zehenspitzen, um das Hineingleiten in einen Schuh zu erleichtern. Der biegsame Sohlenabschnitt kann zu diesem Zweck beispielsweise mehrlagig ausgeführt sein, wovon eine erste Lage zur Verbindung mit dem unbiegsamen Schaftabschnitt dient, z.B. einstückig mit dem darunter verlaufenden biegsamen Schaftabschnitt verbunden ist, wie oben erwähnt, und eine zweite Lage, die vorzugsweise dünner ausgeführt ist als die erste, sozusagen als Schuheinlage dient. In diesem Fall erstreckt sich die erste Lage des biegsamen Sohlenabschnitts von der Ferse bis zum Fußgewölbe und die zweite Lage entweder vom Fußgewölbe oder ebenfalls von der Ferse bis zu den Zehenspitzen. Vorzugsweise ist die zweite Lage des biegsamen Sohlenabschnitts zwischen dessen erster Lage und dem unbiegsamen Sohlenabschnitt angeordnet und besonders bevorzugt mit Letzterem fest verklebt. Die beiden Lagen des biegsamen Sohlenabschnitts können ebenfalls miteinander verklebt sein, sind aber vorzugsweise vernietet, da mittels dieser Nietverbindung gleichzeitig auch der unbiegsame Sohlenabschnitt mit der ersten Lage des biegsamen verbunden werden kann.

Neben dem biegsamen Sohlenabschnitt können natürlich auch sämtliche andere Abschnitte von Schaft und Sohle mehrlagig ausgeführt sein und sind dies vorzugsweise auch. Beispielsweise umfasst der biegsame Schaftabschnitt vorzugsweise mehrere Lagen, wovon jeweils eine an der Innenseite und eine an der Außenseite des unbiegsamen Schaftabschnitts angeordnet und mit diesem verbunden, vorzugsweise verklebt, ist. So ist beispielsweise in bevorzugten Ausführungsformen die an der Außenseite des unbiegsamen Schaftabschnitts vorgesehene Lage des biegsamen Schaftabschnitts an der plantaren (Unter-)Seite der Orthese in distaler Richtung zu den Zehen hin verlängert, um auf einstückige Weise den biegsamen Sohlenabschnitt, oder zumindest eine Lage desselben, zu bilden, während eine innen am unbiegsamen Schaftabschnitt positionierte Lage des biegsamen Schaftabschnitts nur im Bereich oberhalb der Ferse vorgesehen sein kann.

Besonders bevorzugt ist jedoch der unbiegsame Schaftabschnitt sowohl innen als auch außen mit jeweils zumindest einer Lage des biegsamen Schaftabschnitts verklebt, die in jenen Bereichen, die vom unbiegsamen Schaftabschnitt nicht abgedeckt werden direkt miteinander verbunden, vorzugsweise verklebt oder verschweißt, sind.

Weiters besteht auch der unbiegsame Sohlenabschnitt in bevorzugten Ausführungsformen der Erfindung aus mehreren Lagen, beispielsweise indem er an der Oberseite einer unbiegsamen Platte eine hautfreundliche und/oder rutschfeste Schicht aufgebracht ist.

In besonders bevorzugten Ausführungsformen weisen der zweite und der dritte Riemen im Wesentlichen die gleiche Länge auf, so dass es unerheblich ist, welcher der beiden Riemen zum Umschlingen der Knöchel und welcher zum Umschlingen des Fußes verwendet wird. In diesem Fall ist der als dritter Riemen dienende Riemen zweimal um den Fuß zu schlingen, um die beiden Teile des dritten Klettverschlusses miteinander zu verbinden.

Der zweite und der dritte Riemen können zudem einstückig ausgebildet sein, so dass ein durchgehender Riemen, der an den beiden Enden sowie jeweils auf einem Viertel der Länge, von den Enden weg gerechnet, entsprechende Klettverschlussteile aufweist, mit dem unbiegsamen Sohlenabschnitt verbunden ist. Dies ist bei der Herstellung der erfindungsgemäßen Orthese gegenüber der Verwendung getrennter zweiter und dritter Riemen von Vorteil - speziell, wenn die Verbindung mittels Vernieten erfolgt. Außerdem kann am vorderen Ende des unbiegsamen Sohlenabschnitts ein Verbindungselement vorgesehen sein, an dem der zweite und der dritte Riemen jeweils, z.B. lateral, befestigt, z.B. damit vernäht, sind.

Es sei darauf hingewiesen, dass der Schutzumfang der beiliegenden Ansprüche nicht nur auf die darin explizit genannten Klettverschlüsse beschränkt sein soll. Die Nennung Letzterer soll vor allem der Definition der jeweiligen Länge der drei Riemen diesen. Solange jedoch die Wirkung der Erfindung, d.h. die in einer einzigen Orthese kombinierte Funktion einer Sprunggelenksorthese, einer Fußorthese und einer Schuhanziehhilfe, nicht beeinträchtigt wird, d.h. solange die Positionierbarkeit und somit die Funktionsweise der drei Riemen nicht eingeschränkt werden, sind auch Kombinationen aus Haken und Ösen oder Schnürbändern im Sinne der Erfindung als gleichwertige Verschlüsse zu Klettverschlüssen anzusehen.

Freilich erhöhen Klettverschlüsse den Biedienungskomfort für einen Träger der erfindungsgemäßen Orthese, was speziell bei eingeschränkter Beweglichkeit desselben von großer Bedeutung sein kann. Darüber hinaus vereinfachen sie die Reinigung der Orthese, zumal Haken und Ösen beispielsweise eine Waschmaschinenreinigung mitunter verunmöglichen können, und sind gegeüber Haken, Ösen und Schnürbändern eher vor Beschädigung, z.B. durch Riss, Ablösung oder Verbiegen, gefeit.

Nichtsdestotrotz können anstelle der beiden Teile eines Klettverschlusses auch an einer ihrer Positionen eine Öse und an der anderen ein Haken vorgesehen sein, oder es können am Ende eines Riemens zwei Schnürbänder befestigt sein, die nach gegenläufigem Herumführen, z.B. um den Unterschenkel, miteinander verknotbar sind.

Eine bevorzugte Ausführungsform der Orthese gemäß vorliegender Erfindung ist weiters dadurch gekennzeichnet, dass der Schaft im Bereich der Fersenrückseite eine Aussparung aufweist, was vor allem die Bewegungsfreiheit bei Reklination und den Tragekomfort erhöht.

Weiters ist die erfindungsgemäße Orthese bezüglich weiterer Teile, wie z.B. zusätzlichen Versteifungen oder Stützelementen, nicht eingeschränkt, solange die oben angeführte Wirkung nicht beeinträchtigt wird.

Auch das Material der einzelnen Teile der erfindungsgemäßen Orthese ist nicht speziell eingeschränkt, solange es die geforderten Grundvoraussetzungen "biegsam" bzw. "unbiegsam" erfüllt, und kann durchaus aus den üblicherweise für bekannte Orthesen eingesetzten Materialien ausgewählt sein. Aus Kostengründen wird die Wahl in der Regel aus diversen Kunststoffen mit entsprechenden Eigenschaften getroffen werden, obwohl im Einzelfall z.B. auch Leder, Stoff, Metall oder sogar Holz zum Einsatz kommen können, Letztere natürlich für die unbiegsamen Abschnitte von Schaft und Sohlenelement, obgleich auch diese vorzugsweise aus unbiegsamen Kunststoffen, wie z.B. Ebonit (Hartgummi) oder (carbon)faserverstärkten Kunststoffen, gefertigt werden. Die innerste von mehreren Lagen des biegsamen Schaftabschnitts kann zudem beispielsweise aus einem geschäumten Kunststoff bestehen, um den Tragekomfort zu erhöhen, usw.

Die Dimensionen der erfindungsgemäßen Orthese sind ebenfalls nicht speziell eingeschränkt, sondern hängen von den Körpermaßen des beabsichtigten Trägers ab, insbesondere vom Umfang der Wade, des Unterschenkels und des Fußes sowie von der Schuhgröße. So können verschiedene Versionen für schlanke und (stark) übergewichtige Erwachsene, für Kinder verschiedener Altersstufen bzw. kleinwüchsige Erwachsene angeboten werden.

Bei der in den folgenden Figuren dargestellten Ausführungsform beträgt etwa die Gesamtlänge des Sohlenelements rund 26,5 cm, was in Europa etwa einer Schuhgröße von 42 entspricht; die Maximalhöhe des Schafts beträgt rund 24 cm, die Maximalbreite des unbiegsamen Schaftabschnitts (unterhalb der Ferse) rund 8 cm, und die Länge der drei Riemen rund 23 cm für den ersten Riemen bzw. jeweils rund 50 cm für den zweiten und dritten Riemen. Die dargestellte Ausführungsform ist somit für einen durchschnittlichen männlichen Erwachsenen konzipiert, kann aber flexibel an individuelle Bedürfnisse und Körpermaße angepasst werden.

### KURZBESCHREIBUNG DER ZEICHNUNGEN

Nachstehend wird die vorliegende Erfindung unter Bezugnahme auf die beiliegenden Darstellungen der oben erwähnten bevorzugten Ausführungsform der Orthese näher beschrieben. Diese zeigen im Einzelnen Folgendes:
- Fig. 1: eine perspektivische Vorderansicht;
- Fig. 2: eine perspektivische Hinteransicht;
- Fig. 3: eine perspektivische Seitenansicht;
- Fig. 4: eine Seitenansicht;
- Fig. 5: eine Seitenansicht in gegenüber Fig. 4 veränderter Position;
- Fig. 6: eine Unteransicht; und
- Fig. 7: eine Draufsicht von oben auf das Sohlenelement.

### DETAILLIERTE BESCHREIBUNG DER ERFINDUNG

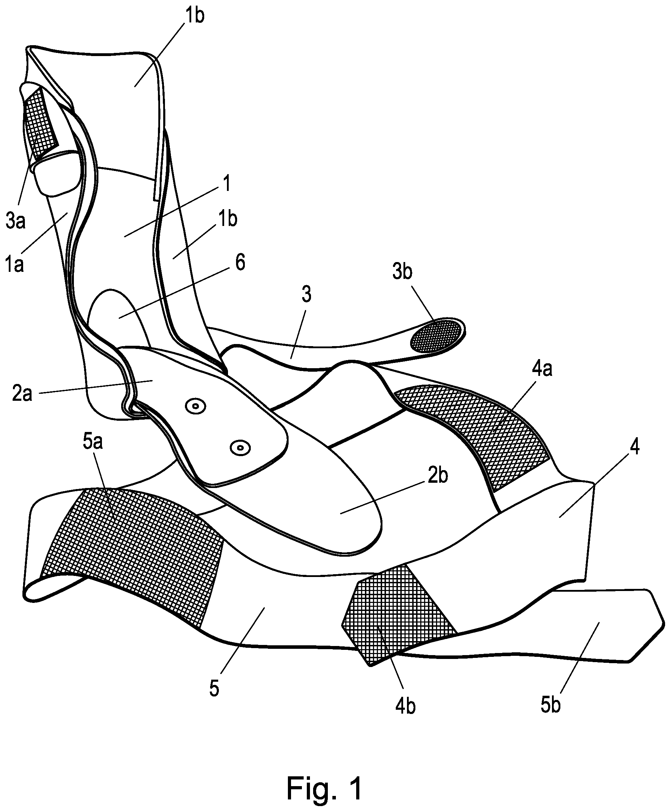

Von den beiliegenden Zeichnungen zeigt Fig. 1 eine perspektivische Vorderansicht einer bevorzugten Ausführungsform der erfindungsgemäßen Orthese. Diese umfasst einen Schaft 1, von dem in der Figur ein unbiegsamer Abschnitt 1 a und zwei Lagen 1 b eines biegsamen Abschnitts zu erkennen sind. Die obere dieser beiden Lagen 1 b ist an der Innenseite der anderen als Polsterung im distalen Wadenbereich vorgesehen. Wie anhand des horizontalen Abstands zwischen dem Abschnitt 1 a und der unteren Lage 1 b zu erkennen ist, würde die erfindungsgemäße Orthese einen durchschnittlich ausgebildeten Unterschenkel eines Trägers nicht zur Gänze umschließen, sondern einen Spielraum frei lassen.

Weiters ist ein Sohlenelement 2 in Form eines unbiegsamen Abschnitts 2a und eines biegsamen Abschnitts 2b dargestellt. Details von Schaft und Sohle sind den nachfolgenden Figuren entnehmbar.

In Fig. 1 ist weiters ein seitlich am distalen Ende des Schafts befestigter erster Riemen 3 mit zwei Teilen 3a und 3b eines zugehörigen Klettverschlusses dargestellt, wovon sich der Teil 3a im Bereich des proximalen Rands und kontralateral zum Befestigungspunkt des Riemens am Schaft und der zweite Teil 3b am Ende des Riemens 3 befinden. Auf der vom Träger der Orthese aus gesehen linken Seite der Sohle ist an dieser ein zweiter Riemen 4 befestigt und auf der rechten Seite ein dritter Riemen 5, die jeweils zwei Teile eines Klettverschlusses umfassen, und zwar jeweils auf etwa halber Länge (4a, 5a) und am Ende (4b, 5b) des Riemens 4 bzw. 5.

Die Anordnung und das Zusammenwirken der drei Riemen 3, 4 und 5 stellt einen wesentlichen Teil der Erfindung dar, da bei Verwendung der erfindungsgemäßen Orthese Riemen 3 quer über den tibialen Bereich des Unterschenkels geführt wird und so den Schaft am Bein fixiert, Riemen 4 quer über den Rist und um den Unterschenkel herum, über beide Knöchel, geführt wird, um die Verbindung zwischen Bein und Fuß zu stabilisieren, und Riemen 5 um den Fuß herum geführt wird und so die Sohle am Fuß fixiert.

Mit Bezugszeichen 6 ist weiters eine Ausnehmung des Schafts im Bereich der Ferse gekennzeichnet.

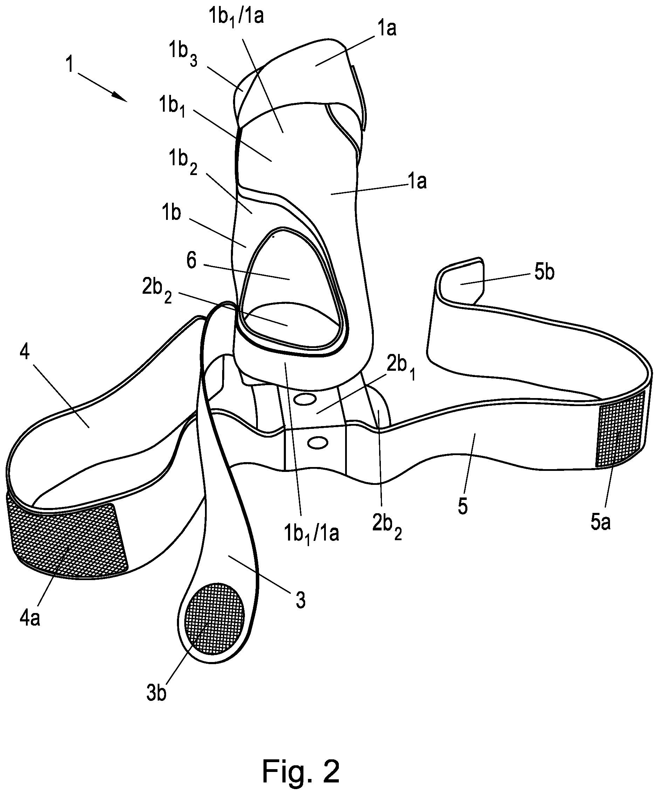

Fig. 2 zeigt eine perspektivische Hinteransicht der Orthese bei abgewinkelter Sohle, in der gleiche Bezugszeichen wie in Fig. 1 und den übrigen Figuren natürlich gleiche Elemente bezeichnen. In Fig. 2 sind vor allem die biegsamen Abschnitte 1b und 2b von Schaft bzw. Sohle detaillierer dargestellt. Konkret sind drei Teile bzw. Lagen 1b₁, 1b₂ und 1b₃ des biegsamen Schaftabschnitts 1 b und zwei Teile bzw. Lagen 2b₁ und 2b₂ des biegsamen Sohlenabschnitts 2b zu erkennen. Der biegsame Schaftabschnitt 1b₁ bedeckt dabei den größten Teil des unbiegsamen Schaftabschnitts 1 a, der von einer plantaren Position unterhalb der Ferse, über den rechten Knöchel aufwärts bis zum Wadenansatz und fibular bis etwa in die Mitte des Unterschenkels reicht. Jene Bereiche, in denen die Lage 1b₁ des biegsamen Schaftabschnitts den unbiegsamen Schaftabschnitt 1 a abdeckt, sind mit 1b₁/1a gekennzeichnet, d.h. die Lage 1b₁ des biegsamen Schaftabschnitts ist an der Außenseite des unbiegsamen Schaftabschnitts 1 a vorgesehen, erstreckt sich fibular (an der Rückseite des Unterschenkels) aber weiter als dieser.

An der Innenseite des unbiegsamen Schaftabschnitts 1 a sind hingegen zwei weitere Lagen 1b₂ und 1b₃ des biegsamen Schaftabschnitts vorgesehen, wovon eine Lage 1b₂ in der linken Hälfte des Unterschenkels auch direkt mit der Innenseite der biegsamen Schaftabschnittslage 1b₁ in Kontakt steht. Die dritte Lage 1b₃ des biegsamen Schaftabschnitts ist ihrerseits an der Innenseite der zweiten Lage 1b₂ vorgesehen, allerdings - wie dies aus Fig. 1 erkennbar ist - nur in deren oberem Bereich zur Verlängerung des Schafts bis zum Wadenansatz und zur Polsterung desselben, um schmerzhaften Druck des unbiegsamen Schaftabschnitts 1 a auf die Wade zu vermeiden.

Weiters ist an der Unterseite der Orthese zu erkennen, dass die Lage 1b₁ is des biegsamen Schaftabschnitts in eine erste Lage 2b1 des biegsamen Abschnitts 2b des Sohlenelements übergeht, d.h. einstückig damit ausgebildet ist. In der in Fig. 2 gezeigten Position mit hochgehobener Ferse ist die zweite Lage 2b₂, die sich von der Fersenausnehmung 6 plantar über die gesamte Sohle bis zu den Zehenspitzen erstreckt, abgeknickt, als würde ein Träger der Orthese "auf den Zehenspitzen", d.h. auf Zehen und Fußballen stehen.

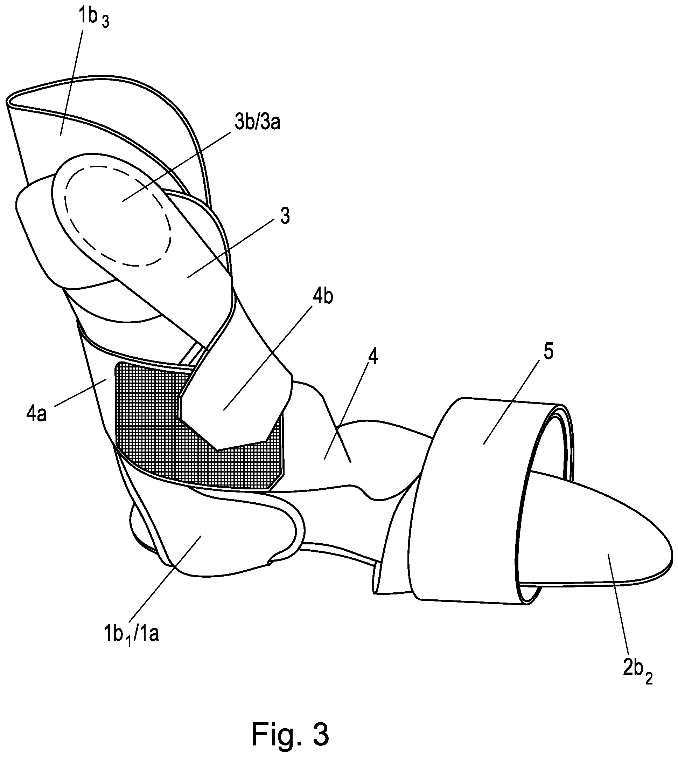

Fig. 3 ist eine perspektivische Seitenansicht der erfindungsgemäßen Orthese mit geschlossenen Klettverschlüssen der in die beim Tragen der Orthese gegebenen Position aller drei Riemen 3, 4 und 5. Wie zu erkennen ist, überlagert der quer über den Rist und um die Knöchel herum geschlungene Riemen 4 dabei den Riemen 3, der den Schaft am Unterschenkel fixiert, und verhindert dadurch ein unbeabsichtigtes Öffnen des Klettverschlusses 3a, 3b des Letzteren. Riemen 5 ist um den Fuß herum geschlungen.

Aufgrund der gleichen Länge der beiden Riemen 4 und 5 wird Letzterer zweimal um den Fuß herum geführt, bis die beiden Teile 5a und 5b des (in Fig. 3 nicht zu erkennenden) Klettverschlusses miteinander zur Deckung gebracht werden. Diese gleiche Länge der Riemen 4 und 5 bewirkt jedoch in vorteilhafter Weise, dass beide Riemen für beide Funktionen verwendet werden können, d.h. dass es für die Funktionsweise der Erfindung keine Rolle spielt, welcher der beiden Riemen um die Knöchel bzw. um den Fuß geschlungen wird. Die bringt für einen Träger der Orthese einen höheren Bedienungskomfort mit sich, was speziell bei eingeschränkter Beweglichkeit (z.B. aufgrund von Verletzung oder Alter) von Vorteil ist.

Wie zuvor in Fig. 2 ist mit 1b₁/1a jener Teil des Schafts gekennzeichnet, wo eine Lage 1 b des biegsamen Schaftabschnitts den unbiegsamen Schaftabschnitts 1 a abdeckt. In analoger Weise ist der geschlossene Klettverschluss des Riemens 3 mit 3b/3a bezeichnet.

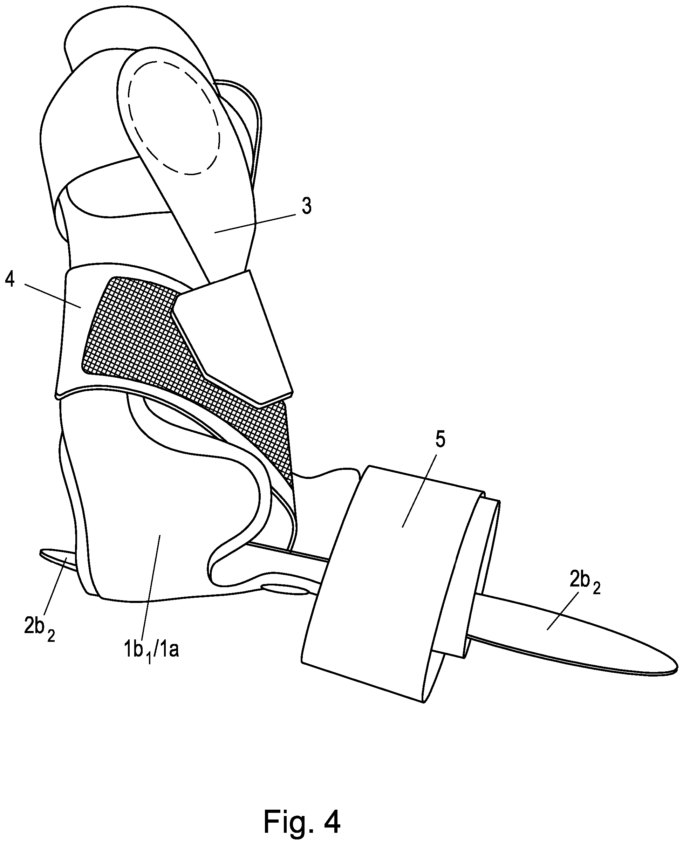

Fig. 4 ist eine Seitenansicht als Draufsicht auf die erfindungsgemäße Orthese von rechts, in der gut zu erkennen ist, dass sich das Sohlenelement 2 (genauer gesagt eine Lage 2b₂ des biegsamen Abschnitts der Sohle) über die gesamte Länge der Sohle des Trägers, unter anderem auch fibular durch die Fersenausnehmung hindurch bis unter die Ferse erstreckt.

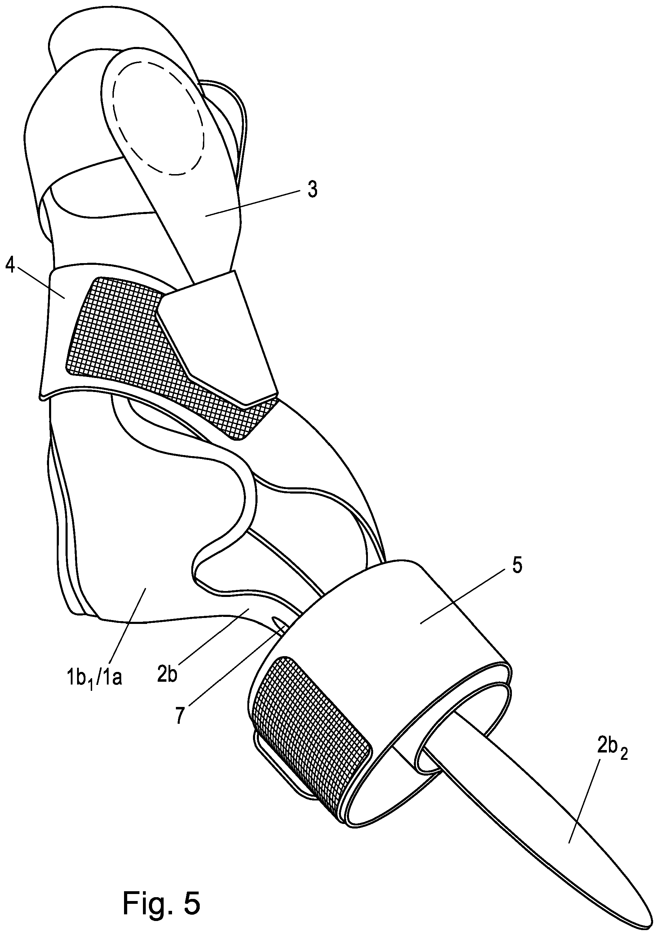

Fig. 5 ist eine zu Fig. 4 analoge Seitenansicht, allerdings in veränderter Position der Orthese, nämlich mit abgewinkelter Stellung des Sohlenelements 2. Diese wird in dieser Ausführungsform der Erfindung dadurch ermöglicht, dass die Lage 1b₁ des biegsamen Schaftabschnitts, die auch plantar unterhalb der Ferse den unbiegsamen Schaftabschnitt 1 a abdeckt, distal über Letzteren hinaus verlängert ist und dadurch nahtlos in eine Lage 2b₁ des biegsamen Sohlenabschnitts 2b übergeht, d.h. damit einstückig ausgeführt ist.

Diese Einstückigkeit der Lagen 1b₁ und 2b₁ der biegsamen Schaft- und Sohlenabschnitte stellt in dieser Ausführungsform der Erfindung auch die einzige Verbindung zwischen dem Schaft und der Sohle dar. Dadurch wird ein Verkippen des Sohlenelements relativ zum Schaft, genauer gesagt relativ zum unbiegsamen Schaftabschnitt 1 a, ermöglicht. Gleichzeitig wird bei einer solchen Streckbewegung des Fußes der Riemen 4 gestrafft, der auf diese Weise die Fußstellung stabilisiert und die Reklination des Fußes unterstützt.

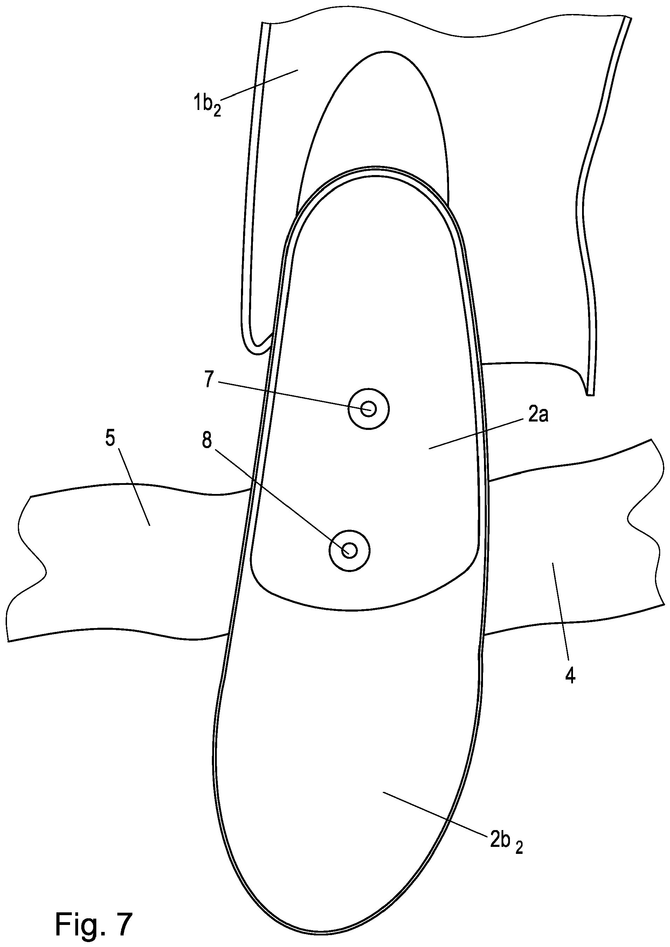

Die Niete 7 hält dabei die beiden Sohlenabschnitte, die (in dieser Ausführungsform) aus insgesamt drei Lagen bestehen, zusammen, da sie nacheinander durch die zuunterst befindliche Lage 2b₁ des biegsamen Sohlenabschnitts, die darüber befindliche Lage 2b₂ des biegsamen Sohlenabschnitts und schließlich durch den unbiegsamen Sohlenabschnitt 2a, wie in Fig. 1 und später in Fig. 7 zu erkennen, hindurch verläuft.

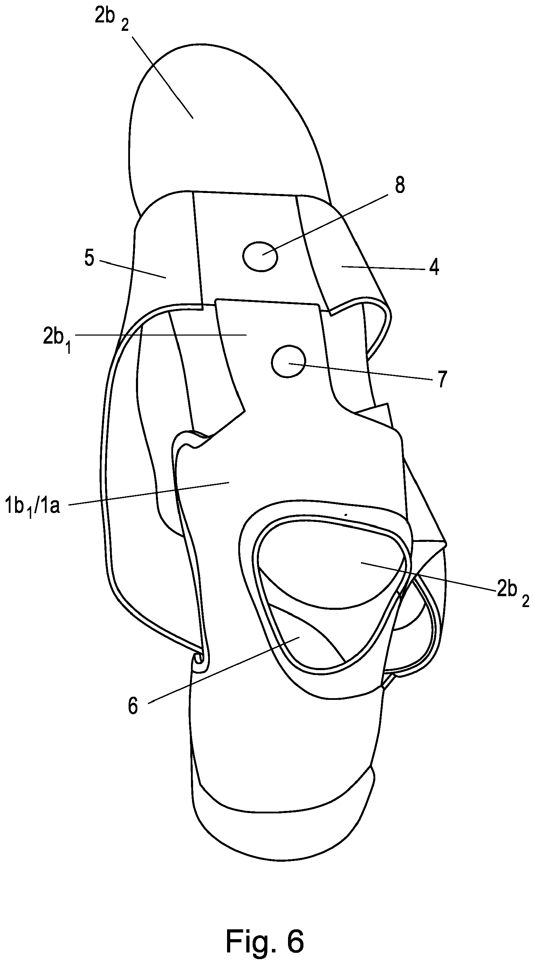

Fig. 6 ist eine perspektivische Unteransicht der Orthese mit geschlossenen Klettverschlüssen, in der zu erkennen ist, dass die Lage 2b₁ des biegsamen Sohlenabschnitts unterhalb des Fußgewölbes endet. Davor befindet sich der Befestigungspunkt der Riemen 4 und 5 mittels der Niete 8. Diese Niete 8 verläuft somit, von unten gesehen, nacheinander durch die beiden Riemen 4 und 5, oder durch einen einzigen, durchgehenden Riemen, dessen Hälften die Riemen 4 und 5 bilden, oder auch durch ein gesondertes Verbindungsstück, an dem die beiden Riemen 4 und 5 befestigt, z.B. vernäht, sind, danach durch die Lage 2b₂ des biegsamen Sohlenabschnitts und schließlich durch den unbiegsamen Sohlenabschnitt 2a, wie in Fig. 1 und später in Fig. 7 zu erkennen, hindurch.

Die Lage 2b₁ des biegsamen Sohlenabschnitts kann sich zwar auch bis zu diesem Befestigungspunkt 8 vorwärts erstrecken, was mitunter die Stabilität der Sohle erhöhen würde. Gleichzeitig würde dies allerdings auch die Dicke der Sohle an dieser Stelle vergrößern, was für den Orthesenträger unangenehm sein könnte, da sich diese Stelle bereits relativ nahe dem Fußballen befindet.

Fig. 7 ist schließlich eine Draufsicht von oben auf das Sohlenelement mit geöffneten Riemen und Klettverschlüssen, in der nach Fig. 1 erneut speziell der (in dieser Ausführungsform transparente) unbiegsame Sohlenabschnitt 2a sowie die Position der Nieten 7 und 8 gut zu erkennen ist. Weiters ist hier die Innenseite der Lage 1b₂ des biegsamen Schaftabschnitts zu sehen, die vorzugsweise aus Rauleder, Stoff oder einem ähnlichen Material besteht, das sich bei direktem Kontakt mit der Haut gut anfühlt. Zu diesem Zweck kann an der Innenseite der Lage 1b₂ auch eine weitere Lage des biegsamen Schaftabschnitts vorgesehen sein.

## Patentansprüche

1. Sprunggelenksorthese, umfassend: einen länglichen Schaft, der von der Ferse bis über den Knöchel verläuft und tibial offen ist, ein mit dem Schaft verbundenes, die Fußsohle zumindest teilweise abdeckendes Sohlenelement, einen ersten Riemen zur Befestigung des Schafts am Unterschenkel und einen zweiten Riemen zur Befestigung des Sohlenelements am Fuß,
**dadurch gekennzeichnet, dass**:
a) der Schaft (1) und das Sohlenelement (2) jeweils einen unbiegsamen Abschnitt (1 a, 2a) und einen biegsamen Abschnitt (1 b, 2b) umfassen,
wobei der unbiegsame Schaftabschnitt (1a) sich von einer plantaren Position unterhalb der Ferse lateral über einen Knöchel hinaus aufwärts erstreckt und im Bereich seines proximalen Randes einen Teil (3a) eines ersten Klettverschlusses aufweist und der biegsame Schaftabschnitt (1b) sich kontralateral von der Ferse über den anderen Knöchel hinaus aufwärts erstreckt,
wobei der unbiegsame Sohlenabschnitt (2a) sich von der Ferse bis zum Fußgewölbe erstreckt und der biegsame Sohlenabschnitt (2b) sich zumindest vom Fußgewölbe in Richtung der Zehen erstreckt;
b) der erste Riemen (3) am distalen Rand des biegsamen Schaftabschnitts (1b) befestigt ist, an seinem Ende den zweiten Teil (3b) des ersten Klettverschlusses aufweist und ausreichende Länge besitzt, um die beiden Teile (3a, 3b) des ersten Klettverschlusses miteinander verbinden zu können;
c) der zweite Riemen (4) am distalen Ende des unbiegsamen Sohlenabschnitts (2a) befestigt ist und zwei Teile (4a, 4b) eines zweiten Klettverschlusses umfasst, wobei ein Teil (4a) des zweiten Klettverschlusses am Ende des zweiten Riemens (4) vorgesehen ist und der andere Teil (4b) in einem mittleren Bereich und auf der gegenüberliegenden Seite des zweiten Riemens (4) vorgesehen ist,
wobei der zweite Riemen (4) ausreichende Länge aufweist, dass die beiden Teile (4a, 4b) des zweiten Klettverschlusses miteinander verbindbar sind, wenn der zweite Riemen (4) quer über den Fußrücken und um beide Knöchel herum geführt wird; und
d) am distalen Ende des unbiegsamen Sohlenabschnitts (2a) auch ein dritter Riemen (5) befestigt ist, der zwei Teile (5a, 5b) eines dritten Klettverschlusses umfasst,
wobei ein Teil (5a) des dritten Klettverschlusses am Ende des dritten Riemens (5) vorgesehen ist und der andere Teil (5b) in einem mittleren Bereich und auf der gegenüberliegenden Seite des dritten Riemens (5) vorgesehen ist,
wobei der dritte Riemen (5) ausreichende Länge aufweist, dass die beiden Teile (5a, 5b) des dritten Klettverschlusses miteinander verbindbar sind, wenn der dritte Riemen (5) um den Fuß herum geführt wird.

2. Orthese nach Anspruch 1, **dadurch gekennzeichnet, dass** der unbiegsame Schaftabschnitt (1a) mit dem biegsamen Sohlenabschnitt (2b) oder der biegsame Schaftabschnitt (1 b) mit dem unbiegsamen Sohlenabschnitt (2a) verbunden ist.

3. Orthese nach Anspruch 1 oder 2, **dadurch gekennzeichnet, dass** der biegsame Schaftabschnitt (1b) und zumindest ein Teil des biegsamen Sohlenabschnitts (2b) einstückig ausgeführt sind.

4. Orthese nach einem der Ansprüche 1 bis 3, **dadurch gekennzeichnet, dass** der unbiegsame Schaftabschnitt (1 a) eine solche Breite aufweist, dass er fibular etwa den halben Unterschenkel abdeckt.

5. Orthese nach einem der vorangegangenen Ansprüche, **dadurch gekennzeichnet, dass** der biegsame Schaftabschnitt (1b) eine solche Breite aufweist, dass er tibial etwa den halben Unterschenkel abdeckt.

6. Orthese nach einem der vorangegangenen Ansprüche, **dadurch gekennzeichnet, dass** der biegsame Sohlenabschnitt (2b) sich bis zu den Zehenspitzen ersteckt.

7. Orthese nach einem der vorangegangenen Ansprüche, **dadurch gekennzeichnet, dass** der zweite und der dritte Riemen (4, 5) im Wesentlichen die gleiche Länge aufweisen.

8. Orthese nach einem der vorangegangenen Ansprüche, **dadurch gekennzeichnet, dass** der zweite und der dritte Riemen (4, 5) einstückig ausgebildet sind.

9. Orthese nach einem der vorangegangenen Ansprüche, **dadurch gekennzeichnet, dass** der Schaft (1) im Bereich der Fersenrückseite eine Aussparung (6) aufweist.

## Claims

1. An ankle orthosis, comprising: a longitudinal shaft which extends from the heel over the ankle and is open at the side of the tibia; a sole element connected to the shaft and at least partially covering the sole of the foot, a first strap for fixing the shaft to the lower leg and a second strap for fixing the sole element to the foot, **characterized in that**:
a) said shaft (1) and said sole element (2) each comprise an inflexible section (1 a, 2a) and a flexible section (1b, 2b),
said inflexible shaft section (1a) extending from a plantar position below the heel laterally upwards over and above one ankle and having one part (3a) of a first hook and loop fastener in the area of its proximal edge and said flexible shaft section (1 b) extending contralaterally from the heel upwards over and above the other ankle,
said inflexible sole section (2a) extending from the heel to the arch of the foot and said flexible sole section (2b) extending at least from the arch of the foot towards the toes;
b) said first strap (3) is fixed to the distal edge of the flexible shaft section (1 b), has the second part (3b) of the first hook and loop fastener at its end and is sufficiently long so that the two parts (3a, 3b) of the first hook and loop fastener can be joined to one another;
c) said second strap (4) is fixed to the distal end of the inflexible sole section (2a) and comprises two parts (4a, 4b) of a second hook and loop fastener,
one part (4a) of said second hook and loop fastener being provided at the end of said second strap (4) and the other part (4b) being provided in a middle part and on the opposite side of said second strap (4),
said second strap (4) being sufficiently long so that the two parts (4a, 4b) of the second hook and loop fastener can be joined to one another when the second strap (4) is laid across the dorsum of the foot and around both ankles; and
d) a third strap (5) is fastened to the distal end of said inflexible sole section (2a), said third strap comprising two parts (5a, 5b) of a third hook and loop fastener,
one part (5a) of said third hook and loop fastener being provided at the end of said third strap (5) and the other part (5b) being provided in a middle part and on the opposite side of said third strap (5),
said third strap (5) being sufficiently long so that the two parts (5a, 5b) of said third hook and loop fastener can be joined to one another when the third strap (5) is laid around the foot.

2. The orthosis according to claim 1, **characterized in that** said inflexible shaft section (1 a) is connected to said flexible sole section (2b) or said flexible shaft section (1b) is connected to said inflexible sole section (2a).

3. The orthosis according to claim 1 or claim 2, **characterized in that** said flexible shaft section (1b) and at least part of said flexible sole section (2b) are integrally formed.

4. The orthosis according to any one of the claims 1 to 3, **characterized in that** said inflexible shaft section (1a) is sufficiently broad to cover approximately half the fibular side of the lower leg.

5. The orthosis according to any one of the preceding claims, **characterized in that** said flexible shaft section (1b) is sufficiently broad to cover approximately half the tibial side of the lower leg.

6. The orthosis according to any one of the preceding claims, **characterized in that** said flexible sole section (2b) extends to the tips of the toes.

7. The orthosis according to any one of the preceding claims, **characterized in that** the second and third straps (4, 5) are of essentially identical length.

8. The orthosis according to any one of the preceding claims, **characterized in that** the second and the third straps (4, 5) are integrally formed.

9. The orthosis according to any one of the preceding claims, **characterized in that** said shaft (1) has an opening (6) in the area of the back of the heel.

## Revendications

1. Chevillère, comprenant: une tige longitudinale s'étendant du talon au travers de la malléole, son côté tibial étant ouvert; un élément de semelle lié à ladite tige et couvrant la plante du pied au moins en partie; une première attache pour attacher la tige au bas de la jambe et une deuxième attache pour attacher ledit élément de semelle au pied,
**caractérisée en ce que**:
a) ladite tige (1) et ledit élément de semelle (2) comprennent respectivement une section rigide (1a, 2a) et une section souple (1b, 2b),
ladite section rigide (1a) de la tige s'étendant d'une position plantaire au-dessous du talon latéralement vers le haut au-delà d'une malléole et ayant une partie (3a) d'une première bande crochet et boucle à l'endroit de son bord proximal et ladite section souple (1b) de la tige s'étendant controlatéralement du talon vers le haut au-delà de l'autre malléole,
ladite section rigide (2a) de la semelle s'étendant du talon jusqu'à la voûte plantaire et ladite section souple (2b) de la semelle s'étendant au moins de la voûte plantaire vers les orteils;
b) ladite première attache (3) est attachée au bord distal de la section souple (1b) de la tige, présente une deuxième partie (3b) de la première bande crochet et boucle à son bout et est suffisamment longue pour que les deux parties (3a, 3b) de la première bande crochet et boucle puissent être reliées;
c) ladite deuxième attache (4) est attachée au bout distal de la section rigide (2a) de la semelle et comprend les deux parties (4a, 4b) d'une deuxième bande crochet et boucle,
une partie (4a) de ladite deuxième bande crochet et boucle étant prévue au bout de la deuxième attache (4) et l'autre partie (4b) étant prévue dans une section intermédiaire et sur le côté opposé de la deuxième attache (4),
ladite deuxième attache (4) étant suffisamment longue pour que les deux parties (4a, 4b) de la deuxième bande crochet et boucle puissent être reliées quand la deuxième attache (4) est mise en travers du cou-de-pied et autour les deux malléoles; et d) une troisième attache (5), comprenant les deux parties (5a, 5b) d'une troisième bande crochet et boucle, est attachée au bout distal du section rigide (2a) de la semelle,
une partie (5a) de ladite troisième bande crochet et boucle étant prévue au bout de la troisième attache (5) et l'autre partie (5b) étant prévue dans une section intermédiaire et sur le côté opposé de la troisième attache (5),
ladite troisième attache (5) étant suffisamment longue pour que les deux parties (5a, 5b) de la troisième bande crochet et boucle puissent être reliées quand la troisième attache (5) est mise autour le pied.

2. Chevillère selon la revendication 1, **caractérisée en ce que** la section rigide (1a) de la tige est reliée à la section souple (2b) de la semelle ou la section souple (1b) de la tige est reliée à la section rigide (2a) de la semelle.

3. Chevillère selon la revendication 1 ou 2, **caractérisée en ce que** la section souple (1 b) de la tige et au moins une partie de la section souple (2b) de la semelle sont faites en une seule pièce.

4. Chevillère selon une des revendications 1 à 3, **caractérisée en ce que** la section rigide (1 a) de la tige présente une telle largeur qu'elle couvre environ la moitié du côté du péroné du bas de jambe.

5. Chevillère selon une des revendications précédentes, **caractérisée en ce que** la section souple (1 b) de la tige présente une telle largeur qu'elle couvre environ la moitié du côté tibial du bas de jambe.

6. Chevillère selon une des revendications précédentes, **caractérisée en ce que** la section souple (2b) de la semelle s'étend jusqu'aux pointes des pieds.

7. Chevillère selon une des revendications précédentes, **caractérisée en ce que** la deuxième et la troisième attache (4, 5) sont essentiellement également longues.

8. Chevillère selon une des revendications précédentes, **caractérisée en ce que** la deuxième et la troisième attache (4, 5) sont faites en une seule pièce.

9. Chevillère selon une des revendications précédentes, **caractérisée en ce que** la tige (1) a un évidement (6) à l'endroit de l'arrière du talon.
